**Europäisches Patentamt**

**European Patent Office**

**Office européen des brevets**

(19)

(11) Publication number: **0 064 788**

**A1**

(12)

# EUROPEAN PATENT APPLICATION

(21) Application number: **82200499.0**

(22) Date of filing: **26.04.82**

(51) Int. Cl.³: **G 01 S 13/58**
**G 01 S 13/52, A 61 B 5/10**

(30) Priority: **27.04.81 NL 8102059**

(43) Date of publication of application:
**17.11.82 Bulletin 82/46**

(84) Designated Contracting States:
**AT BE CH DE FR GB IT LI LU NL SE**

(71) Applicant: **RIJKSUNIVERSITEIT LEIDEN**
**Stationsweg 46 P.O. Box 9500**
**NL-2300 RA Leiden(NL)**

(72) Inventor: **Kemp, Bastiaan**
**Sweelincklaan 20**
**NL-2394 GP Hazerswoude Rijndijk(NL)**

(72) Inventor: **Buruma, Onno Jacob Sytze**
**Wilhelminapark 3**
**NL-2342 AD Oegstgeest(NL)**

(72) Inventor: **Franzen, Jan Marius**
**Mozartstraat 12**
**NL-2324 XW Leiden(NL)**

(72) Inventor: **Roos, Raymundus Albertus Christianus**
**Brederostraat 24**
**NL-2332 BB Leiden(NL)**

(74) Representative: **van der Beek, George Frans, Ir. et al,**
**Nederlandsch Octrooibureau P.O. Box 29720**
**NL-2502 LS The Hague(NL)**

(54) System and method for observing and quantifying body movements.

(57) System for observing and quantifying body movements, comprising a Doppler radar unit transmitting on the one hand high frequency electromagnetic waves in the direction of the body on body part of the person in which said body movements are appearing and receiving on the other hand reflected electromagnetic waves and converting said waves into signal trains. Said system comprises a circuit recovering a signal from the eventually amplified signal trains, the amplitude of which signal is a function of the amplitude and/or the frequency of the incoming signal train.

System and method for observing and quantifying body movements.

The invention relates to a system for observing and quantifying body movements, comprising a Doppler radar unit transmitting on the one hand high frequency electromagnetic waves in the direction of the body or body part of the person in which said body movements are appearing and receiving on the other hand reflected electromagnetic waves and converting said waves into signal trains.

Such a system is known from the French patent 2.141.047. The system from said French patent comprises a Doppler radar unit of which the output signal first of all is delivered to a bandfilter/amplifier and thereafter is supplied to an accumulator. The output of the accumulator is connected to an alarm circuit. As long as signals are delivered at the output of the Doppler radar unit, that means as long as with a certain regularity body movements are observed, the accumulator in the form of a capacitor will maintain a sufficient charge so that the alarm circuit is not activated. However, if said body movements vanish, then the Doppler radar unit will supply no further output signals and than the capacitor in the ac- cumulator is able to discharge after which the alarm is activated.

By means of said system only an indication is provided about the fact that during a predetermined time period, the discharging period of the accumulator capacitor, no movement is detected. Howeverit is impossible to quantatively judge body movements by means of said system.

In the medical world there is a strong need for systems and methods for judging especially involuntary movements in a quantitive way, on the one hand to get a clear picture of the course of the underlying desease as well as to judge the influence of medicins on the course of the underlying desease. The involuntary movements, which are especially meant, are e.g. appearing as result of extra pyramidal syndroms, epilepsia and ataxia.

From "Involuntary movement disorders" by J.S.Cooper, 1969, Hoeber Medical Division, Harper and Row, New York a cinematografical technic for registering involuntary movements is known, which technic, however, is very labour intensive and therefore very expensive. Furthermore said technic is only applicable in a very restricted way, because only the movements of a restricted number of points of the body of a patient can be measured.

An electromyografical method, which can be applicated simultaneously to several muscle groups is amongst others known from "The Diagnosis and treatment of athetosis and dystonia" by T.J.Putnam, published in Journal of Bone and Joint Surgery, nr. 21, 1939, pages 948-957. By means of said method, however, only a very restricted number of muscles can be examined. Therefore said method only delivers a very small contribution to the investigation of involuntary movements.

A further development of this technique, by means of which deviations originating from a tracing movement carried out as result of an instruction, can be analysed is described in "Measurement of involuntary arm movement in athetotic patients" by P.D. Neilson, published in Journal of Neurology, Neurosurgery and Psychiatry, Nr.37, 1974, pages 171-177. Besides the above indicated restriction of the electromyografic method said further development has also the disadvantage that it is not possible to measure the amount of movement commotion in rest. Also an analysis of involuntary movements in the face area is not possible with this method.

None of the known methods provides the possibility to quantitively judge involuntary movements in such a sense that the juration of the movement as well as the speed components during said movement and furthermore the size of the body part involved into said movement or taken into account when making said judgement.

An object of the invention is now to provide a method and a system by means of which involuntary movements can be judged in a very simple way on the basis of the above-mentioned criteria without annoyance for the patient.

Said object is according to the invention reached in a system of the above mentioned type, in that said system comprises a circuit recovering a signal from the eventually amplified signal trains, the amplitude of which signal is a function of the amplitude as well as the frequency of the incoming signal train. Said system is especially suited for quantitively judging extra pyramidal symdrons and ataxia.

Said object is according to the invention also reached with a system of the above mentioned type in that said system comprises a circuit recovering a signal from the eventually amplified signal trains, the amplitude of which is a function of the frequency of the incoming signal train. Said embodiment of the system is especially suited for quantitively judging body movements resulting from epilepsia.

A preferred embodiment of the system according to the invention is characterized in that the recovered signal is processed into an average value by means of an averaging circuit.

Such an averaging unit provides a discrete value forming a clear quantative indication about the rate and strength of the observed movements over a predetermined time period. On the basis of such a quantative indication a doctor is able to determine if e.g. a therapy is successful or not.

A further preferred embodiment of the system according to the invention is characterized in that said circuit comprises a module in which the incoming signal trains are subjected to a Fourier transformation process.

4

By means of the Fourier transformer it is possible to distinguish between the various speed components.

In a further embodiment said circuit comprises a module in which the incoming signal trains are differentiated. By differentating the signal trains the amplitudes in the output signal trains are multiplied by the frequency of the various components.

In a preferred embodiment of the system, used for recovering a signal of which the amplitude is a funcion of only the frequency of the incoming signal train said recovering circuit comprises a phase lock loop module or a comparator followed by a frequency to voltage convertor.

If thereafter the signal is rectified or squared and the average of the outcoming signal trains is determined by means of an integrator unit, then the result will be an ultimate value which is depending on the number of movements, the duration of said movements, the speed of said movements and the surface extent of the body parts involved in said movements.

To eliminate low fysiological movement components, e.g. caused by the movement of the body or body part as result of the breathing function, and furthermore eliminate fast fysiological movement components, e.g. caused by the eye flicker movements, it is preferred that a band filter is inserted between the Doppler radar unit and said circuit which band filter only passes frequencies within a predetermined frequency band.

Because furthermore the system according to the invention is one time used with a patient showing relatively weak movements and is another time used with a patient showing relatively strong movements, it is preferred to insert an amplifier with a controllable amplification factor into the system.

The above mentioned object of the invention is furthermore reached by means of a method for quantitively judging involuntary body movements e.g. caused by extra pyramidal syndroms, epilepsia and ataxia, characterized by the use as described above, by means of which high frequency electromagnetic waves are transmitted in the direction of the body or body part of the person in which said body movements are appearing and by means of which the reflected electromagnetic waves are received and converted into a signal train of which the length equals the duration of the corresponding movement, of which the power is proportional to the size of the body or body part involved in said movement and/or of which the frequency is proportional to the speed components in the observed movement, from which signal trains signals are recovered giving a good indication about the strength of the movement, which recovered signals are eventually averaged over a predetermined time period.

Although the related involuntary movements usually have a random movement direction, it is possible that there is a dominating di-rection into said movement. To be sure that also movements which are directed parallel to the wave front of the transmitted electromagnetic waves will contribute to the signal trains according to a preferred method two or three mutually perpendicular means of electromagnetic waves are transmitted in the direction of the body or body part of the person involved in said movements.

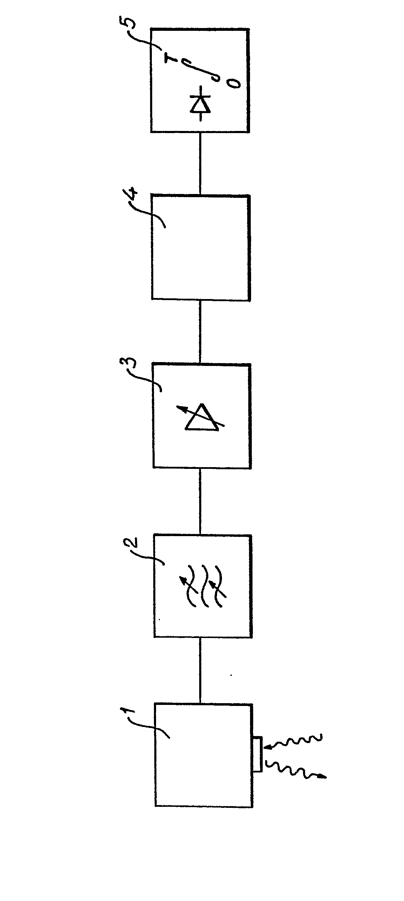

The invention will be discussed in more detail hereafter referring to the figure in which a block diagram of a system according to the invention is illustrated.

The block diagram in the figure comprises a Doppler radar unit 1, a band filter 2, a controllable amplifier 3, a converter 4 and an integrator unit 5.

The Doppler radar unit 1 may be embodied as a commercially availa-ble Doppler radar module, comprising a transmitter by means of which a beam of electromagnetic waves is transmitted in the direction of the patient to be investigated or in the direction of a body part of said patient. Because of the high water content of the human body the electromagnetic waves are in principle reflected by the body and received by the receiver present in the Doppler radar module and thereafter by means of a mixing stage converted into an electrical signal. If the body of the patient is completely in rest then the Doppler radar receiver will not deliver any AC voltage at his output. Every movement of the patient, however, results into a signal train of which the length is equal to the duration of the corresponding movement, the frequency is linear proportional to the speed components in the observed movement and the power is proportional to the size of the body or body part involved into said movement. Each body part situated within reach of the Doppler radar receiver is additively contributing a component to this signal, so that the power of the signal trains at the output of the Doppler radar receiver is linearly proportional to the size of the radiated surface

The signal trains delivered by said Doppler radar module 1 are supplied to a band filter 2 in which undesired frequency components can be removed. In general e.g. a lower frequency limit will be determined such that frequencies corresponding to the slow movements of body parts resulting from the breathing function are filtered out of the signal trains. Also frequencies resulting from slow voluntary movements, which a patient e.g. is instructed to carry out are preferably filtered out by means of this band filter 2. In general said breathing movements and voluntary movements carried out on instruction or much slower than the involuntary movements as result of extrapyramidal syndroms, epilepsia and ataxia, which should be judged by means of the system according to the invention.

The output signal of the band filter 2 is supplied to a controlable amplifier 3. Dependent on the expected or during a premeasurement observed signal amplitudes said amplifier 3 is adjusted to such an amplification factor that on the one hand the measuring circuit as a whole is not saturated whereas on the other hand a relatively high output signal is delivered to the converter 4 so that the sensitivity of the whole system in each case will become as high as possible.

The output signal of the controllable amplifier 3 is supplied to a convertor 4. The aim of said convertor is to make the amplitude in the signal trains dependent on the frequencies found into said signal trains. In a first embodiment of the invention a differentiator module is used into said convertor 4 for differentiating the incoming signal trains. The result thereof is that all the amplitude components are multiplied by the signal frequency , so that the amplitudes in the output signal trains are linearly proportional to the speed of the movement. The output signals of said differentiator are delivered to a rectifier or squaring circuit inside the convertor 4 delivering an output signal which is dependent onto the surface extent of the body parts involved in said movements as well as dependent onto the speeds of the observed movements.

The output signal of the convertor 4 is thereafter averaged during a predetermined time period in a unit 5. In a preferred embodiment said unit comprises a circuit functioning as an integrator 5 bymeans of which the incoming signal trains are integrated during a predetermined time period. Said time periode may be in the order of a number of minutes. All the signal trains appearing within this time period are integrated together and are contributing to the ultimate integration value which is dependent onto the duration of the movements, the speed components in the observed movements and the extent of the body surface involved into said movements.

In case the system according to the invention is used for delivering a signal of which the amplitude is a function of only the frequency of the incoming train then the unit 4 can comprise a phase locked loop module or a comparator followed by a frequency to voltage convertor. Both possible circuits are generating an output signal of which the amplitude is only a function of the frequency of the incoming signal.

The system which is described as example, function as follows. The patient is sitting or lying such that the Doppler radar module is able to radiate electromagnetic waves in the direction of the patient. If the distance between the Doppler radar and the patient is large, e.g. about 2 meter, then a larger part of the body of the patient can be observed for involuntary movements. However, it is also possible to radiate a much smaller part of the patient, e.g. only his face by means of the Doppler radar by choosing the distance between the Doppler radar and the concerned part of the patient's body smaller. The selected Doppler radar should have a low radiation power density exposure beneath the international security standards. The Doppler radar used into the prototype causes at the minimum application distance of 50 cm a radiation power density $<$ 0,01 mW/cm$^2$ which is a value underneath all the now pending security standards.

Because the movements of the patient are exclusively observed by means of the Doppler radar module the patient is not hindered by sticking electrodes or detectors of another kind. In some cases the patient will be requested to carry out prescribed movements during the application of the method. However, the invention can also be used on a patient which is not able to carry out the instructed movements.

If necessary the investigation can be repeated and registrations during a longer time period are possible. In most cases, however, a registration of some minutes (2 to 5 minutes) will be sufficient.

In comparison with the prior art methods the method and the use of the system according to the invention has a very low labour intensity.

In the above described embodiment of the invention the convertor 4 comprised a differentiator module. It is, however, also possible to use in this convertor 4 a module in which the incoming signal trains are subjected to a Fourier transformer to distinguish the various speed components in the signal trains. In that case the output signals related to each frequency are preferably per frequency averaged into the averaging unit.

In this embodiment the convertor 4 and eventually also the averaging unit 5 can be realized by means of a microcomputer.

Using a differentiator module in the convertor 4 results, however, into a very simple convertor because only some operational amplifiers are necessary to realize such a differentiator unit. The exact embodiment of the differentiator is considered as known to the expert in this field.

The integration module used in the averaging unit 5 in a preferred embodiment can be realized by means of a microprocesser which is controlled by a special programm such that the incoming signals are integrated with an integration time which is adjustable between 10 and 990 seconds in steps of 10 seconds. However, it will be clear that also other limit values and steps values can be selected. Not only the converter 4 and the averaging unit 5 can be realized by means of digital methods, also the amplifier 3 and the band filter 2 can be realized by digital means, in which case between the Doppler radar module 1 and the band filter 2 an analog/digital converter should be inserted.

The method according to the invention, as described up to now, is in principle direction-dependent. Movements in the direction of the Doppler radar module will be observed optimally, however, movements perpendicular to the radiation beam will be observed into a less extent or will not be observed at all. In general the movement directions of the involuntary movements caused by extra pyramidal syndroms and epilepsia will be randomly distributed, so that said direction sensitivity, taking into account a sufficiently number of movements, (the integration time is chosen sufficiently large) does not play any role. In other cases, e.g. in the case of ataxia, whereby involuntary movements should be judged quantitively have to be judged said direction sensitivity can play a role. In that case it is preferred to transmit two or three mutually perpendicular beams of electromagnetic waves in the direction of the body or body part of the person in which said involuntary body movements are appearing. That can be realized by using three different Doppler radar modules which are transmitting three mutually perpendicular beams of electromagnetic waves. The signal trains delivered at the outputs of said three Doppler radar modules can be processed each into a separate chain consisting of the described units 2,3,4, 5 whereby the output indications at the outputs of the averaging units 5 can be averaged in turn or can be combined in another way. However, it is also possible to combine the output signals of the Doppler radar modules into a previous stage and to use one processing chain.

On the other hand it is furthermore possible to use reflectors to radiate mutually perpendicular beams of electromagnetic waves starting from only one Doppler radar module. In that case the whole processing chain can be embodied simplier.

Although there are theoretically several possibilities for calibrating a system according to the invention, the realisation of most of these possibilities will be very difficult or impossible in practice.

According to the invention a pendulum is now used for calibrating the system. A pendulum has the advantage that it is an object of known size and shape, carrying out simple to prescribe standard movements which are reproducable in a normal hospital with very simple means. Preferably the pendulum is embodied as an elongated thin wire or stem carrying a metal spherical body.

11

## CLAIMS

1. System for observing body movements, comprising a Doppler radar unit transmitting on the one hand high frequency electromagnetic waves in the direction of the body or body part of the person in which said body movements are appearing and receiving on the other hand reflected electromagnetic waves and converting said waves into signal trains, <u>characterized in that</u> said system comprises a circuit recovering a signal from the eventually amplified signal trains, the amplitude of which signal is a function of the amplitude as well as the frequency of the incoming signal train.

2. System for observing body movements, comprising a Doppler radar unit transmitting on the one hand high frequent electromagnetic waves in the direction of the body or body part of the person in which said body movements are appearing and receiving on the other hand reflected electromagnetic waves and converting said waves into signal trains, <u>characterized in that</u> said system comprises a circuit recovering a signal from the eventually amplified signal trains, the amplitude of which is a function of the frequency of the incoming signal train.

3. System according to claim 1 or 2, <u>characterized in that</u> the recovered signal is processed into an average value by means of an averaging circuit.

4. System according to claim 1, <u>characterized in that</u> said circuit comprises a module in which the incoming signal trains are subjected to a Fourier transformation process.

5. System according to claim 1, <u>characterized in that</u> said circuit comprises a module in which the incoming signal trains are differentiated.

6. System according to calim 2, <u>characterized in that</u> said circuit comprises a phase lock loop.

7. System according to claim 2, characterized in that said circuit comprises a comparator followed by a frequency to voltage convertor.

8. System according to claim 5,6 or 7, characterized in that said circuit furthermore comprises a squaring circuit.

9. System according to one of the claims 5,6 or 7, characterized in that said circuit comprises a rectifier.

10. System according to one of the preceding claims, characterized in that the averaging circuit comprises an integrator.

11. Method for calibrating the system described in one of the preceding claims, characterized in that said system is calibrated by means of a pendulum

12. Method for quantatively judging involuntary body movements, e.g. caused by extra pyramidal syndroms epilepsia and ataxia, characterized by the use of a system according to one of the preceding claims, by means of which high frequency electromagnetic waves are transmitted in the direction of the body or body part of the person in which said body movements are appearing and by means of which the reflected electromagnetic waves are received and converted into a signal train of which the length equals the duration of the corresponding movement, of which the power is proportional to the size of the body or body part involved in said movement and/or of which the frequency is proportional to the speed components in the observed movement, from which signal trains signals are recovered giving a good indication about the strength of the movement, which recovered signals are eventually averaged over a predetermined time period.

13. Method according to claim 12, characterized in that two or three mutually perpendicular beams of electromagnetic waves are transmitted in the direction of the body or body part of the person in which said body movements are appearing.

## EUROPEAN SEARCH REPORT

**European Patent Office**

Application number

EP 82 20 0499

### DOCUMENTS CONSIDERED TO BE RELEVANT

| Category | Citation of document with indication, where appropriate, of relevant passages | Relevant to claim | CLASSIFICATION OF THE APPLICATION (Int Cl. ³) |
|---|---|---|---|
| X | US-A-3 618 084 (R. BALSIGER et al.)<br>* Column 3, lines 3-47; column 5, lines 15-21 *<br><br>--- | 1,2,5 | G 01 S 13/58<br>G 01 S 13/52<br>A 61 B 5/10 |
| A | MEDICAL AND BIOLOGICAL ENGINEERING, vol. 13, no. 2, March 1975, page 317, Stevenage, G.B.<br>J.W. HEAL: "An animal activity monitor using a microwave Doppler system" * Whole article *<br><br>--- | 1,10, 12 | |
| A | FR-A-1 561 844 (ADVANCED DEVICES LABORATORY)<br>* Page 1, line 1 - page 10, line 14 *<br><br>--- | 1,12 | |
| A | MICROWAVE JOURNAL, vol. 21, no. 5, May 1978, pages 69-72, Dedham, USA<br>D.W. GRIFFIN: "MW interferometers for biological studies" * Page 69, the paragraph: "Movement is the basic surface effect"; page 71, the paragraph: "Measurement of small movements of the human body" *<br><br>--- | 1,12 | **TECHNICAL FIELDS SEARCHED (Int. Cl. ³)**<br><br>A 61 B<br>G 01 S |
| A | US-A-3 993 995 (G.S. KAPLAN et al.)<br>* Column 1, line 5 - column 2, line 43 *<br><br>---<br><br>-/- | 1,5,8 | |

The present search report has been drawn up for all claims

| Place of search | Date of completion of the search | Examiner |
|---|---|---|
| THE HAGUE | 04-08-1982 | MARCHAU M.F. |

0064788

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl. ³) |
|---|---|---|---|
| A | US-A-3 242 486 (P.I. CORBELL) * Column 1, line 9 - column 2, line 34 * | 1,3,10 | |
| D | FR-A-2 141 047 (PEAK TECHNOLOGIES) * Page 2, line 2- page 3, line 9; page 8, lines 32-39; page 14, lines 19-33 * | 1,10 | |

TECHNICAL FIELDS SEARCHED (Int. Cl. ³)

The present search report has been drawn up for all claims

| Place of search | Date of completion of the search | Examiner |
|---|---|---|
| THE HAGUE | 04-08-1982 | MARCHAU M.F. |